# EUROPÄISCHE PATENTSCHRIFT

(11) **EP 1 605 837 B1**
(45) Veröffentlichungstag und Bekanntmachung des Hinweises auf die Patenterteilung: **14.04.2010**
(21) Anmeldenummer: 04706650.1
(22) Anmeldetag: 30.01.2004
(51) Int. Cl.: A61B 17/16, B23B 31/00, A61C 1/14

(54) **KUPPLUNG FÜR EIN CHIRURGISCHES DREHANTRIEBS-HANDSTÜCK**
COUPLING FOR A SURGICAL ROTARY DRIVE TOOL HOLDER
ACCOUPLEMENT POUR UN PORTE-OUTIL CHIRURGICAL A MECANISME D'ENTRAINEMENT

(30) Priorität: 15.03.2003 DE 10311455
(43) Veröffentlichungstag der Anmeldung: 21.12.2005
(73) Patentinhaber: Aesculap AG, 78532 Tuttlingen (DE)
(72) Erfinder: BLUST, Edgar, 78126 Königsfeld (DE); MÜLLER, Thomas, CH-4512 Bellach (CH)
(74) Vertreter: Boehme, Ulrich
(86) Internationale Anmeldenummer: PCT/EP2004/000853
(87) Internationale Veröffentlichungsnummer: WO 2004/082490

(56) Entgegenhaltungen:
- DE-A- 3 934 610
- US-A- 2 751 229
- US-A- 5 222 956
- US-A- 5 928 241
- US-A- 5 989 257
- US-A1- 2002 151 902

## Beschreibung

Die Erfindung betrifft eine Kupplung für ein chirurgisches Drehantriebs-Handstück mit einem in dem Handstück gelagerten und von diesem drehend angetriebenen Werkzeug mit einer hülsenförmigen, drehangetriebenen Aufnahme im Handstück, in die ein Schaft des Werkzeuges unter Ausbildung einer formschlüssigen Drehmitnahme einschiebbar ist, und mit mindestens einem radial in den Innenraum der Aufnahme einschiebbaren Verriegelungskörper, der in einer eingeschobenen Verriegelungsstellung in einen Rücksprung des Schaftes eingreift und diesen dadurch gegen axiale Verschiebung sichert, während er in einer radial ausgeschobenen Stellung aus dem Rücksprung austritt und damit eine axiale Verschiebung des Schaftes in der Aufnahme ermöglicht.

Chirurgische Drehantriebs-Handstücke werden eingesetzt, um Bohrer, Fingerfräser oder ähnliche schnell laufende schaftförmige Werkzeuge anzutreiben, die zur Bearbeitung von Zähnen, Knochen etc. benötigt werden. Die Werkzeuge müssen dabei nach Bedarf auswechselbar sein, und es ist erwünscht, daß dies durch einfaches Einschieben und Herausziehen unter gleichzeitiger Herstellung bzw. Aufhebung einer Drehverbindung zum Drehantrieb des Handstückes erreicht werden kann.

Es sind derartige Kupplungen bekannt, bei denen die Werkzeuge in eine Aufnahme eingeschoben und dann durch manuelle Betätigung einer Verriegelungseinrichtung in axialer Richtung festgelegt werden. Dies erfordert einen separaten Betätigungsschritt, und bei häufigem Werkzeugwechsel während einer chirurgischen Operation kann dies störend sein.

In der DE 3934610 A1 ist eine gattungsgemäße Kupplung beschrieben, bei der jedoch die Übertragung eines Drehmomentes über radiale Stifte erfolgt, die in eine entsprechende Ausnehmung einer hülsenförmigen Aufnahme eingreifen. In dieser Aufnahme ist ein Stempel axial verschieblich gelagert, der als Auswerfer-Element dient, wenn die axiale Verriegelung des Schaftes eines Werkzeuges in der Aufnahmehülse aufgehoben wird.

Verschiedene Formen des Schaftes im Bereich von das Drehmoment übertragenden Anlageflächen sind weiter aus der US 2002/0151902 A1 bekannt, diese Flächen wirken mit radial verschieblichen, kugeligen Verriegelungskörpern zusammen, die in der rotierenden Aufnahmehülse radial verschieblich gelagert sind.

Es ist daher Aufgabe der Erfindung, eine gattungsgemäße Kupplung so auszugestalten, daß eine Festlegung des in die Kupplung eingesetzten Werkzeuges ohne manuelle Betätigung einer Verriegelungseinrichtung erfolgt.

Diese Aufgabe wird bei einer Kupplung der eingangs beschriebenen Art erfindungsgemäß dadurch gelöst, daß zur Ausbildung einer formschlüssigen Drehmitnahme in der hülsenförmigen Aufnahme ein mit der Hülse rotierender, gegenüber dieser axial verschieblicher Mitnehmer angeordnet ist mit mindestens einer Anlagefläche an seiner dem Werkzeug zugewandten Seite, daß der Mitnehmer unter der Wirkung einer Feder in eine ausgeschobene Stellung verschiebbar ist, in der der oder die Verriegelungskörper an ihm anliegen und dadurch in ihrer Freigabestellung gehalten werden, daß der Mitnehmer mittels des an ihm anliegenden Schaftes des Werkzeuges gegen die Wirkung der Feder in eine eingeschobene Stellung verschiebbar ist, in der der oder die Verriegelungskörper in die Verriegelungsstellung eintreten und den Schaft des Werkzeuges gegen eine axiale Verschiebung sichern können, und daß der Schaft des Werkzeuges in dessen eingeschobener, durch den oder die Verriegelungskörper gegen eine axiale Verschiebung gesicherten Stellung den Mitnehmer gegen die Wirkung der Feder derart in die Aufnahme einschiebt, daß dessen Anlageflächen an Anlageflächen des Werkzeuges unter Ausbildung einer Drehmitnahme anliegen.

Es wird also in der Aufnahme ein Mitnehmer angeordnet, dem eine Doppelfunktion zukommt. Einerseits stellt dieser Mitnehmer die drehfeste Verbindung her zwischen der Aufnahme und dem Schaft des Werkzeuges, andererseits dient der Mitnehmer als Auswerfer für das in die Aufnahme eingesetzte Werkzeug und als Blockiereinrichtung, die die Verriegelungskörper in der Freigabestellung hält, wenn kein Werkzeug in die Aufnahme eingesetzt ist. Werden die Verriegelungskörper freigegeben, so daß sie sich unbehindert radial nach außen bewegen können, dann schiebt der Mitnehmer unter der Wirkung der Feder das Werkzeug aus der Aufnahme hinaus und legt sich dem oder den Verriegelungskörpern so gegenüber, daß diese nicht mehr in die radial innenliegende Verriegelungsstellung gelangen können. Erst wenn der Mitnehmer durch ein in die Aufnahme eingeschobenes Werkzeug wieder aus dieser vorderen Stellung zurückgeschoben wird, können die Verriegelungskörper wieder radial nach innen verschoben werden, allerdings erst dann, wenn der Schaft des Werkzeuges vollständig eingeschoben ist, so daß der Rücksprung des Schaftes den Verriegelungskörpern gegenüberliegt. In dieser Stellung kann der Schaft dann einfach dadurch verriegelt werden, daß die Verriegelungskörper radial nach innen verschoben werden. Dies könnte zwar auch manuell erfolgen, wird aber vorzugsweise durch eine Feder automatisch erreicht, so daß eine manuelle Betätigung einer Verriegelungseinrichtung überflüssig wird. In der eingeschobenen und verriegelten Stellung des Werkzeuges werden die Anlageflächen des Schaftes und die Anlageflächen des Mitnehmers unter der Wirkung der Feder gegeneinander gespannt, so daß dadurch eine sichere Drehmitnahme gewährleistet ist.

Die Anlageflächen werden besonders geschont, wenn eine großflächige Drehmomentseinleitung gewährleistet ist, dies läßt sich beispielsweise dadurch erreichen, daß gemäß einer bevorzugten Ausführungsform die Anlageflächen des Mitnehmers und des Werkzeuges flächig aneinander anliegen.

Es kann aber bei einer abgewandelten Ausführungsform auch vorgesehen sein, daß die Anlageflächen des Mitnehmers und des Werkzeuges linienförmig aneinander anliegen.

Bei einer besonders bevorzugten Ausführungsform ist vorgesehen, daß die Anlageflächen an einem Teil einen Einführtrichter ausbilden und am anderen Teil eine Einführspitze. Grundsätzlich ist es möglich, den Einführtrichter entweder am Schaft des Werkzeuges oder am Mitnehmer vorzusehen und dementsprechend die Einführspitze am jeweilig anderen Teil, bevorzugt wird aber eine Ausgestaltung, bei der die Einführspitze am Schaft des Werkzeuges angeordnet ist und der Einführtrichter an dem Mitnehmer, da auf diese Weise die radiale Ausdehnung des Werkzeugschaftes gering gehalten werden kann. Dies kann bei den sehr hohen verwendeten Drehzahlen von Bedeutung sein.

Günstig ist es, wenn der Mitnehmer und das Werkzeug im Bereich ihrer Anlageflächen spiegelsymmetrisch zu einer Spiegelebene sind, die durch die Drehachse der Aufnahme und durch einen Durchmesser des eingesetzten Werkzeuges aufgespannt wird. Eine besonders vorteilhafte Ausgestaltung ergibt sich, wenn die Aufnahme und das Werkzeug jeweils zwei Anlageflächen aufweisen. Es gibt dann bei einer symmetrischen Ausgestaltung zwei Einkupplungslagen, die durch die Ausgestaltung als Einführtrichter beim Einschieben des Werkzeugschaftes in die Aufnahme zwangsläufig eingenommen werden.

Insbesondere kann vorgesehen sein, daß die Normalenvektoren der beiden Anlageflächen des Werkzeuges und/oder des Mitnehmers parallel zueinander verlaufen, insbesondere in einer Ebene liegen.

Bei einer besonders bevorzugten Ausführungsform sind die beiden Anlageflächen des Mitnehmers und/oder des Werkzeuges exakt oder annähernd V-förmig angeordnet, die Anlageflächen nähern sich also keilförmig einander an. Dadurch ergibt sich eine besonders gute Einführhilfe und Winkelausrichtung zwischen Mitnehmer und Werkzeugschaft.

Bei einer bevorzugten Ausführungsform ist vorgesehen, daß die Anlageflächen des Mitnehmers und/oder des Werkzeuges eben sind. Dies ermöglicht eine flächige Anlage und erleichtert die Herstellung.

Es kann aber auch vorgesehen sein, daß die Anlageflächen des Werkzeuges oder des Mitnehmers eine gebogene Kontur aufweisen. Die gebogene Kontur kann dabei in Längsrichtung der Anlagefläche vorliegen, also in einer Richtung eines Schenkels der V-Form, oder aber auch quer dazu. Bevorzugt wird dabei eine Kontur, bei der die Anlagefläche quer zur Längsrichtung eben ist und in Längsrichtung gebogen.

Dabei kann vorgesehen sein, daß die Kontur im drehachsnahen Bereich konvex ausgebildet und im drehachsfernen Bereich demgegenüber zurückgesetzt ist. Man erhält auf diese Weise eine linienförmige Anlage der Anlageflächen aneinander, insbesondere wenn eine der beiden Anlageflächen eben und die andere gebogen ausgebildet ist.

Die Zurücksetzung kann beispielsweise dadurch geschaffen werden, daß die Kontur im drehachsfernen Bereich konkav ausgebildet ist.

Es kann weiterhin vorgesehen sein, daß der Öffnungswinkel des Einführtrichters im drehachsfernen Bereich größer ist als im drehachsnahen Bereich. Dies erleichtert die Einfuhr und führt dazu, daß die Anlageflächen im drehachsnahen Bereich näher aneinanderliegen, daß also mehr Material für die außenliegenden Anlageflächen zur Verfügung steht.

Bei einer anderen Ausführungsform sind die Anlageflächen parallel zur Drehachse der Aufnahme angeordnet und verlaufen somit auch parallel zueinander. Diesen parallelen Anlageflächen kann eine einführtrichterähnliche Erweiterung zugeordnet sein.

Der den Verriegelungskörper aufnehmende Rücksprung am Schaft des Werkzeuges kann eine Umfangsnut sein.

Da der Schaft und die Mitnahme jedoch in ganz bestimmten relativen Winkelstellungen zueinander stehen, kann vorgesehen sein, daß der den Verriegelungskörper aufnehmende Rücksprung eine quer zur Drehachse verlaufende, geradlinige Nut an der Außenseite des Werkzeuges ist oder sogar eine randseitig geschlossene Vertiefung an der Außenseite des Werkzeuges, insbesondere in Form einer Kugelkalotte. Dadurch erfolgt eine wesentlich geringere Schwächung des Werkzeugschaftes im Bereich des Rücksprunges als bei der Ausbildung als umlaufende Umfangsnut, und dies kann insbesondere bei kleinen Durchmessern des Werkzeugschaftes und bei den notwendigen hohen Drehzahlen von Vorteil sein.

Bei einer bevorzugten Ausführungsform ist der Mitnehmer an seinem dem Werkzeug zugewandten, als Einführtrichter ausgebildeten Ende keilförmig ausgebildet. Dies erleichtert zusätzlich die Einführung des Werkzeuges. Der Keilwinkel kann dabei zwischen 60° und 90° liegen, vorzugsweise liegt er im Bereich von etwa 75°.

Es ist vorteilhaft, wenn der Mitnehmer in der Aufnahme außenseitig einen Rücksprung aufweist, in den der Verriegelungskörper eintaucht, wenn sich der Mitnehmer bei entspannter Feder in seiner ausgeschobenen Stellung befindet. Dadurch ist sichergestellt, daß beim Einführen des Schaftes und beim Anpassen der Winkelstellung von Mitnehmer und Schaft der Mitnehmer noch nicht die Verriegelungskörper freigibt, erst nach dieser die Winkelstellung justierenden Einführung des Schaftes wird der Benutzer durch kräftigeren Druck auf das Werkzeug den Mitnehmer gegen die Wirkung der Feder zurückschieben und dabei auch die Verriegelungskörper aus den außenseitigen Rücksprüngen des Mitnehmers nach außen drängen. Dieser Rücksprung weist dabei normalerweise eine sehr geringe Tiefe aus, so daß dieses Verdrängen der Verriegelungskörper keine allzu großen Kräfte erfordert.

Die Ausschubbewegung des Mitnehmers wird vorzugsweise durch einen Anschlag der Aufnahme begrenzt.

Es kann vorgesehen sein, daß der Mitnehmer einen zu seiner Verschieberichtung parallelen Schlitz aufweist, durch den ein an der Aufnahme festgelegter Mitnahmestift hindurchragt. Dadurch wird der Mitnehmer drehfest mit der Aufnahme verbunden, jedoch axial frei verschieblich. Der Mitnahmestift begrenzt außerdem die axiale Bewegung des Mitnehmers und wirkt somit als Anschlag.

Es ist günstig, wenn der Verriegelungskörper in der eingeschobenen Verriegelungsstellung an der Kante des Rücksprunges punkt- oder linienförmig anliegt, dadurch ergibt sich eine genau definierte Axialstellung des Werkzeuges. Beispielsweise kann der Verriegelungskörper eine Kugel sein und der Rücksprung kann eine bogenförmige Kontur aufweisen, deren Radius geringfügig kleiner ist als der Radius des Verriegelungskörpers. Der kugelförmige Verriegelungskörper liegt dann an der Kante des Rücksprunges an.

Besonders vorteilhaft ist es, wenn der Verriegelungskörper federnd in die Verriegelungsstellung verschoben wird.

Grundsätzlich ist es möglich, nur einen einzigen Verriegelungskörper vorzusehen, es ist aber vorteilhaft, wenn mehrere Verriegelungskörper Verwendung finden, beispielsweise zwei.

Besonders günstig ist es, wenn die Verriegelungskörper Kugeln sind.

Der Verriegelungskörper kann vorzugsweise in einer radialen Öffnung der Aufnahme verschieblich geführt sein.

Es ist weiterhin vorteilhaft, wenn die Aufnahme zur Verschiebung des Verriegelungskörpers von einer Verschiebehülse mit einer Aufgleitfläche für den Verriegelungskörper umgeben ist, die vorzugsweise in Richtung auf ein radiales Einschieben des Verriegelungskörpers federbelastet ist.

Die Aufgleitfläche kann mindestens in einem Teilabschnitt relativ zur Verschieberichtung der Verschiebehülse geneigt sein, durch diese Neigung und die Federbelastung der Verschiebehülse drückt die Verschiebehülse den Verriegelungskörper in dessen Verriegelungsstellung federnd radial nach innen.

Der Schutz erstreckt sich auch auf ein Werkzeug, welches in weiteren Unteransprüchen angegebene Merkmale aufweist, die die Verwendung in der erfindungsgemäßen Kupplung ermöglichen.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht durch ein chirurgisches Drehantriebs-Handstück mit eingesetztem Werkzeug;
- Figur 2:: eine vergrößerte Teilschnittansicht im Bereich der den Schaft des Werkzeuges aufnehmenden Aufnahme des Drehantriebs-Handstückes der Figur 1 bei eingesetztem und gegen axiale Verschiebung gesichertem Werkzeug;
- Figur 3:: eine Ansicht ähnlich Figur 2 mit ausgeschobenem Werkzeug und vorgeschobenem Mitnehmer;
- Figur 4:: eine Draufsicht auf den Schaft eines Werkzeuges in Anlage an einem Mitnehmer gemäß einer ersten bevorzugten Ausführungsform;
- Figur 5:: eine Seitenansicht der Anordnung der Figur 4;
- Figur 6 bis Figur 11:: abgewandelte Ausführungsformen der Verbindungsbereiche zwischen Werkzeugschaft und Mitnehmer;
- Figur 12:: eine vergrößerte Detailansicht des Kupplungsbereiches mit einem kugelförmigen Verriegelungskörper, der an der Kante des Werkzeugrücksprunges anliegt;
- Figur 13:: eine Ansicht ähnlich Figur 4 mit einem keilförmig zugespitzten Mitnehmer und
- Figur 14:: eine Ansicht ähnlich Figur 5 des Mitnehmers der Figur 13.

Das in der Zeichnung dargestellte Handstück 1 umfaßt ein zylindrisches Gehäuse 2 mit einem Anschlußteil 3 an seiner Rückseite und einem sich an der gegenüberliegenden Seite konisch verjüngenden Spitzenteil 4. Über das Anschlußteil 3 wird das Gehäuse 2 in aus der Zeichnung nicht ersichtlicher Weise an einen Antrieb angeschlossen, beispielsweise an einen in einem Gehäuse angeordneten Elektromotor.

Im Innern des zylindrischen Gehäuses 2 ist über zwei Kugellager 5, 6 eine zylindrische Aufnahme 7 in Form einer zum vorderen Ende des Handstückes 1 hin offenen Aufnahmehülse 7 gelagert, die an ihrem dem Anschlußteil 3 zugewandten Ende in aus der Zeichnung nicht ersichtlicher Weise mit dem Drehantrieb des an das Anschlußteil 3 angeschlossenen Motors drehfest verbindbar ist. Die Aufnahme 7 weist einen zum Spitzenteil 4 hin offenen zylindrischen Innenraum 8 auf, dieser ist am gegenüberliegenden Ende durch einen Boden 9 verschlossen. In den Innenraum ist drehfest mit diesem verbunden ein Mitnehmer 10 eingesetzt, der im wesentlichen kreiszylindrisch ausgebildet ist und im Innenraum der Aufnahme 7 an deren Innenwand in Längsrichtung der Aufnahme verschieblich geführt ist. Durch einen Längsschlitz 11 im Mitnehmer 10 tritt ein quer zur Längsrichtung verlaufender, an der Aufnahme 7 festgelegter Stift 12 hindurch, der einerseits den Mitnehmer 10 mit der Aufnahme 7 drehfest verbindet und der andererseits die Längsverschiebung des Mitnehmers 10 begrenzt.

An seinem dem Boden 9 zugewandten Ende stützt sich eine Druckfeder 13 an dem Mitnehmer 10 ab, deren gegenüberliegendes Ende in einer Vertiefung des Bodens 9 aufgenommen ist und somit den Mitnehmer 10 mit einer Federkraft beaufschlagt, die versucht, den Mitnehmer 10 aus der Aufnahme 7 hinauszuschieben. Mit anderen Worten kann der Mitnehmer 10 nur gegen die Wirkung dieser Druckfeder 13 in Richtung auf den Boden 9 verschoben werden.

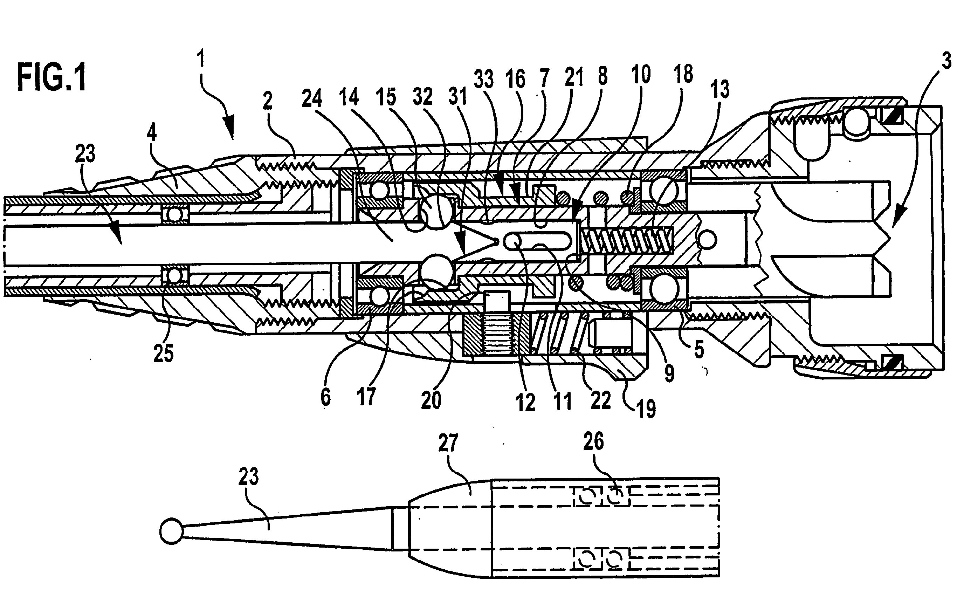

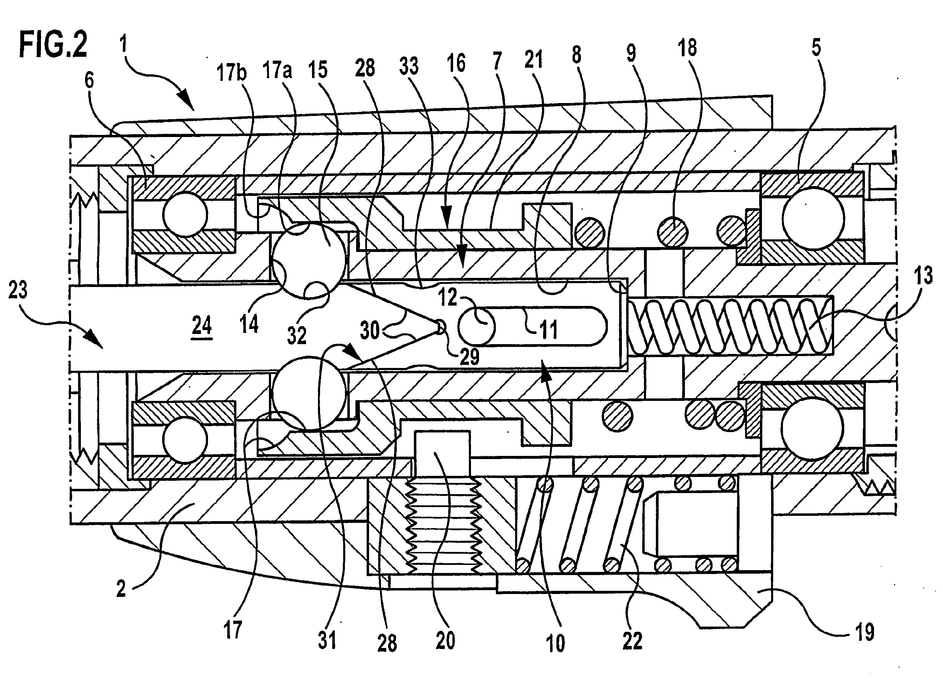

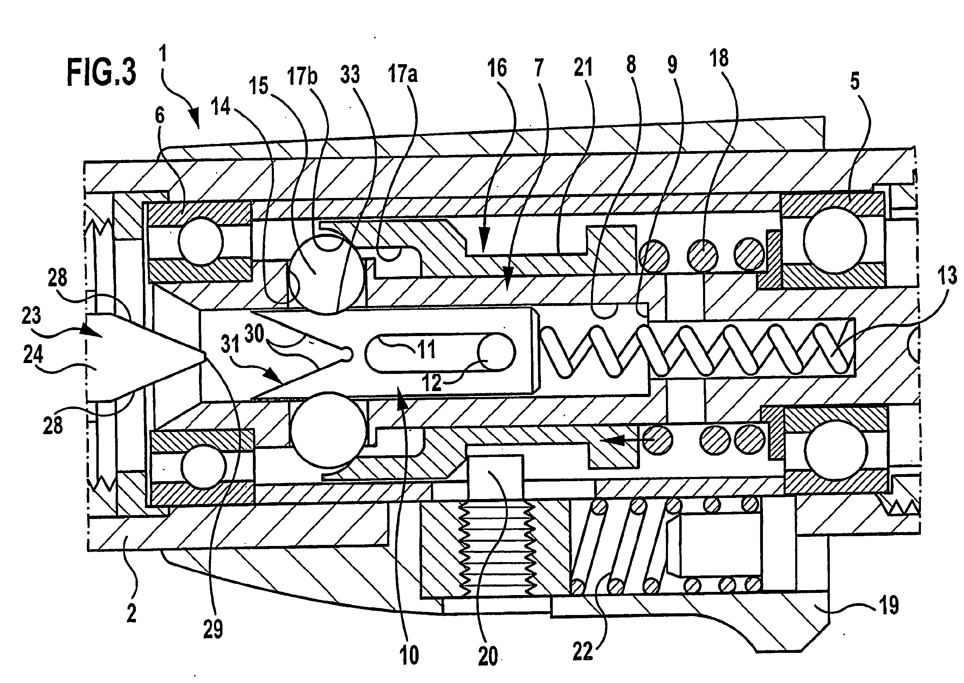

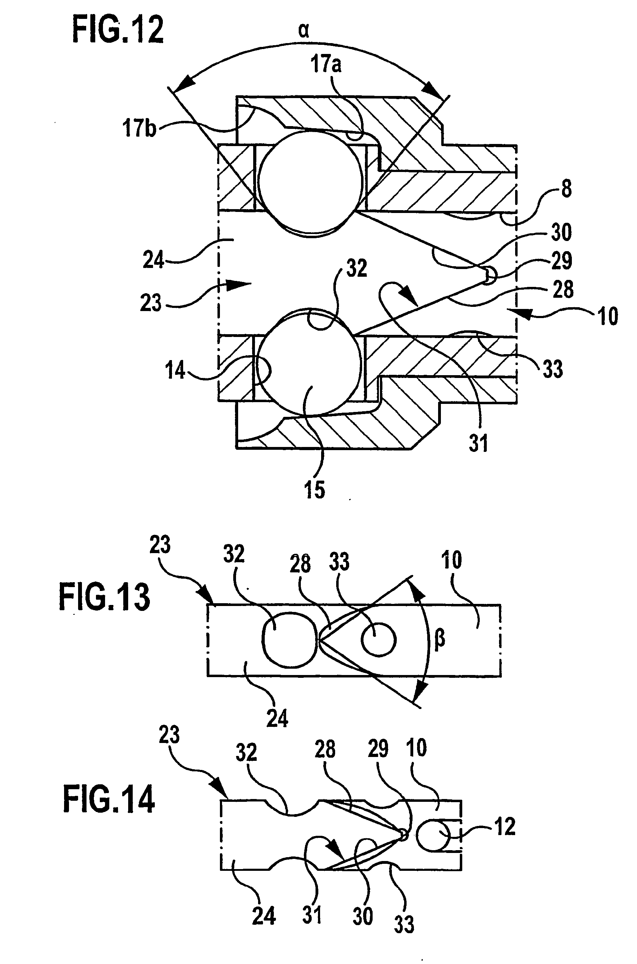

Der Abschnitt 17a der Andruckfläche 17 ist bei dem Ausführungsbeispiel der Figuren 1 bis 3 kreiszylindrisch ausgebildet, beim Ausführungsbeispiel der Figur 12 dagegen geringfügig konisch, so daß dieser Abschnitt gegenüber der Verschieberichtung der Verschiebehülse 16 geneigt verläuft. Dadurch wird der Verriegelungskörper 15 auch in seiner eingeschobenen Verriegelungsstellung nach innen gespannt, da die Verschiebehülse 16 unter der Wirkung der Schraubenfeder 18 nach vorne geschoben wird.

Der Einsatz besteht vorzugsweise aus einem besonders festen Material, beispielsweise aus Hartmetall.

In kurzem Abstand anschließend an das vordere Ende des Mitnehmers 10 befinden sich in der Wand der Aufnahme 7 diametral gegenüberliegend zwei kanalförmige, radiale Durchbrüche 14, in jedem dieser Durchbrüche 14 ist ein kugelförmiger Verriegelungskörper 15 radial verschieblich geführt, so daß die Verriegelungskörper 15 radial in den Innenraum vorstehend in diesen eingeschoben und aus diesem wieder ausgeschoben werden können.

Auf der Außenseite der Aufnahme 7 ist axial verschieblich eine Verschiebehülse 16 gelagert, die die Verriegelungskörper 15 mittels einer ringförmigen Andruckfläche 17 überfängt, die nebeneinander einen kreiszylindrischen und einen schräg abfallenden, gebogenen Abschnitt 17a bzw. 17b aufweist. Verschiebt man die Verschiebehülse 16 auf der Aufnahme 7 in einer Richtung, so werden die Verriegelungskörper 15 an der Andruckfläche 17b anliegend radial nach innen verschoben, bei der Verschiebung der Verschiebehülse in umgekehrter Richtung erhalten die Verriegelungskörper 15 dagegen so viel Spiel, daß sie radial nach außen aus dem Innenraum 8 der Aufnahme 7 herausgeschoben werden können.

Die Verschiebehülse 16 wird mit Hilfe einer die Aufnahme 7 umgebenden Schraubenfeder 18 in eine Verriegelungsstellung gespannt, in der sie die Verriegelungskörper 15 maximal radial nach innen einschiebt. Um die Verriegelungskörper 15 radial nach außen auszuschieben, muß also die Verschiebehülse 16 entgegen der Wirkung der Schraubenfeder 18 auf der Aufnahme 7 verschoben werden.

Die Verschiebung der Verschiebehülse 16 erfolgt mit Hilfe eines auf dem Gehäuse 2 gelagerten und dieses umgebenden Griffteiles 19, welches mit einem Mitnehmerstift 20 durch eine Öffnung des Gehäuses 2 in den Gehäuseinnenraum hineinragt und dort in eine Ringnut 21 auf der Außenseite der Verschiebehülse 16 eintaucht. Der Mitnehmerstift 20 hat dabei gegenüber allen Wänden der Ringnut 21 normalerweise Spiel und wird in dieser Stellung durch eine Schraubenfeder 22 gehalten, die das Griffteil 19 in einer Ruhestellung festlegt, aus der es unter Spannung der Schraubenfeder 22 verschoben werden kann. Beim Verschieben des Griffteiles 19 gelangt dieses an der Seitenwand der Ringnut 21 zur Anlage und nimmt dann die Verschiebehülse 16 mit in die Freigabestellung, in welcher die Verriegelungskörper 15 radial nach außen ausgeschoben werden können.

In den Innenraum 8 der Aufnahme 7 ist von der offenen vorderen Seite des Handstückes 1 durch dessen Spitzenteil 4 hindurch ein schaftförmiges Werkzeug 23 eingeschoben, dessen Schaft 24 einen Außendurchmesser aufweist, der dem Innendurchmesser des Innenraumes 8 entspricht. Dadurch wird dieser Schaft 24 in dem Innenraum 8 quer zu dessen Längsrichtung geführt, eine weitere Führung ergibt sich durch Kugellager 25, 26, die im Inneren des Spitzenteils 4 und in einer sich daran anschließenden hülsenförmigen Verlängerung 27 angeordnet sind.

Der Schaft 24 ist an seinem hinteren Ende bei dem in den Figuren 1 bis 5 dargestellten Ausführungsbeispiel keilförmig zugespitzt und weist zwei ebene Anlageflächen 28 auf, die V-förmig aufeinander zulaufen und eine zentrale Spitze 29 ausbilden. Die Anlageflächen 28 sind dabei so angeordnet, daß deren Normalenvektoren in einer Ebene liegen, die Anlageflächen 28 sind bezüglich einer Mittelebene des Werkzeuges 23 spiegelsymmetrisch, die durch die Drehachse und durch einen Durchmesser des Werkzeuges 23 aufgespannt wird.

Diese Anlageflächen 28 sind komplementär ausgestaltet zu ebenen, keil- oder V-förmig verlaufenden Anlageflächen 30 am Mitnehmer 10, diese Anlageflächen 30 bilden einen keilförmigen Einführtrichter 31 für die Spitze 29 des Werkzeuges 23 aus.

An der Außenseite des Schaftes 24 sind einander gegenüberliegend zwei geradlinige Nuten 32 eingearbeitet, die einen kreisförmigen Querschnitt aufweisen, dessen Durchmesser im wesentlichen dem der kugelförmigen Verriegelungskörper 15 entspricht. Die beiden Nuten 32 verlaufen dabei parallel zu den Ebenen, die durch die Anlageflächen 28 definiert werden.

Im Mitnehmer 10 sind in dessen Außenwand zwei kugelkalottenförmige Vertiefungen 33 geringer Tiefe eingearbeitet, diese liegen einander diametral gegenüber, so daß sie beim Vorschieben des Mitnehmers 10 unmittelbar vor den Durchbrüchen 14 angeordnet sind, so daß die Verriegelungskörper 15 in diese Vertiefungen 33 eintauchen können. Die Tiefe der Vertiefung 33 ist dabei deutlich geringer als die Tiefe der Nut 32 im Schaft 24 des Werkzeuges 23.

Um in das Handstück 1 ein Werkzeug 23 einsetzen zu können, müssen zunächst die Verriegelungskörper 15 in der radial ausgefahrenen Stellung stehen, dies läßt sich dadurch erreichen, daß die Verschiebehülse 16 entgegen der Wirkung der Schraubenfeder 18 zurückgeschoben wird. Dadurch können die Verriegelungskörper 15 radial nach außen geschoben werden, und zwar durch den Mitnehmer 10, der unter der Wirkung der Druckfeder 13 in die vorgeschobene Stellung verschoben wird und dabei die kugelförmigen Verriegelungskörper 15 so weit nach außen verschiebt, bis diese auf seiner Außenseite abrollen oder entlanggleiten. Die Vorschubbewegung des Mitnehmers 10 wird durch den im Längsschlitz 11 geführten Stift 12 begrenzt, und zwar derart, daß bei vollständig vorgeschobenem Mitnehmer 10 die Vertiefungen 33 genau dem Durchbruch 14 gegenüberliegen. Die radial nach außen geschobenen Verriegelungskörper 15 liegen am bogenförmigen Abschnitt 17b der Andruckfläche 17 der Verschiebehülse 16 an und verhindern, daß diese in die vorgeschobene Stellung zurückkehren kann. Andererseits drückt die Andruckfläche 17 mit ihrem bogenförmigen Abschnitt 17b die Verriegelungskörper 15 unter der Wirkung der Schraubenfeder 18 radial nach innen in die Vertiefung 33 hinein. Dadurch wird der Mitnehmer 10 in seiner ausgeschobenen Stellung nicht nur durch die Druckfeder 13 gehalten, sondern zusätzlich auch durch die Verriegelungskörper 15, die nach Art einer elastischen Raste in die Vertiefungen 33 eingreifen.

Schiebt man von der offenen Seite her den Schaft 24 eines Werkzeuges 23 in die Aufnahme 7 hinein, so gelangt die Spitze 29 des Schaftes 24 in den Einführtrichter 31 des Mitnehmers 10. Dabei legen sich die Anlageflächen 28 flächig an die Anlageflächen 30 an, und dies führt zu einer Verdrehung des Werkzeuges 23, bis die Anlageflächen 28 dieselbe Orientierung einnehmen wie die Anlageflächen 30. Bei dieser Winkeljustierung wird der Mitnehmer 10 durch die in die Vertiefungen 33 eintauchenden Verriegelungskörper 15 festgelegt. Allerdings kann der Benutzer diese Festlegung überwinden, wenn er nach erfolgter Winkeljustierung das Werkzeug 23 mit größerer Kraft in die Aufnahme 7 hineindrückt. Dabei werden die Verriegelungskörper 15 radial nach außen gedrückt und verschieben dabei die Verschiebehülse 16 gegen die Wirkung der Schraubenfeder 18 weiter zurück, da sie an dem bogenförmigen Abschnitt 17b der Andruckfläche 17 anliegen. Sobald das Werkzeug 23 so weit eingeschoben ist, daß die Nut 32 den Durchbrüchen 14 und damit den Verriegelungskörpern 15 gegenüberliegt, können diese radial nach innen in die Nut 32 eintreten und geben dadurch die Verschiebehülse 16 frei, die unter der Wirkung der Schraubenfeder 18 in die vorgeschobene Stellung verschoben wird, so daß jetzt der zylindrische Abschnitt 17a der Andruckfläche 17 außenseitig an den Verriegelungskörpern 15 anliegt und verhindert, daß diese radial nach außen verschoben werden können, der Schaft 24 des Werkzeuges 23 ist damit in axialer Richtung im Gehäuse 2 festgelegt.

In dieser festgelegten Stellung des Werkzeuges 23 wird der Mitnehmer 10 durch die Druckfeder 13 gegen den Schaft 24 gespannt, so daß sich im Bereich der Anlageflächen 28 und 30 durch deren flächige Anlage eine zuverlässige Drehmitnahme ergibt, die Drehbewegung der Aufnahme 7 wird also über den Stift 12 auf den Mitnehmer 10 übertragen und vom Mitnehmer 10 auf das Werkzeug 23.

Zur Entnahme des Werkzeuges 23 genügt es, die Verschiebehülse 16 gegen die Wirkung der Schraubenfeder 18 zurückzuziehen, dadurch werden die Verriegelungskörper 15 in radialer Richtung freigegeben, und der Mitnehmer 10 kann nunmehr unter der Wirkung der Druckfeder 13 nach vorne geschoben werden, dabei wird auch das Werkzeug 23 aus der Aufnahme 7 ausgeschoben. In der gleichen Weise, wie oben beschrieben, blockiert dann der Mitnehmer 10 die Verriegelungskörper 15 in der radial ausgeschobenen Stellung, so daß der Werkzeugwechsel in gleicher Weise erfolgen kann, wie oben beschrieben.

Der Mitnehmer hat somit zusätzlich noch die Wirkung eines Auswerfers des in der Aufnahme 7 aufgenommenen Werkzeuges 23.

Bei dem in den Figuren 1 bis 5 dargestellten Ausführungsbeispiel sind die Anlageflächen 28 und 30 eben ausgebildet und liegen flächig aneinander an, die Spitze 29 und der Einführtrichter 31 sind also zueinander komplementär.

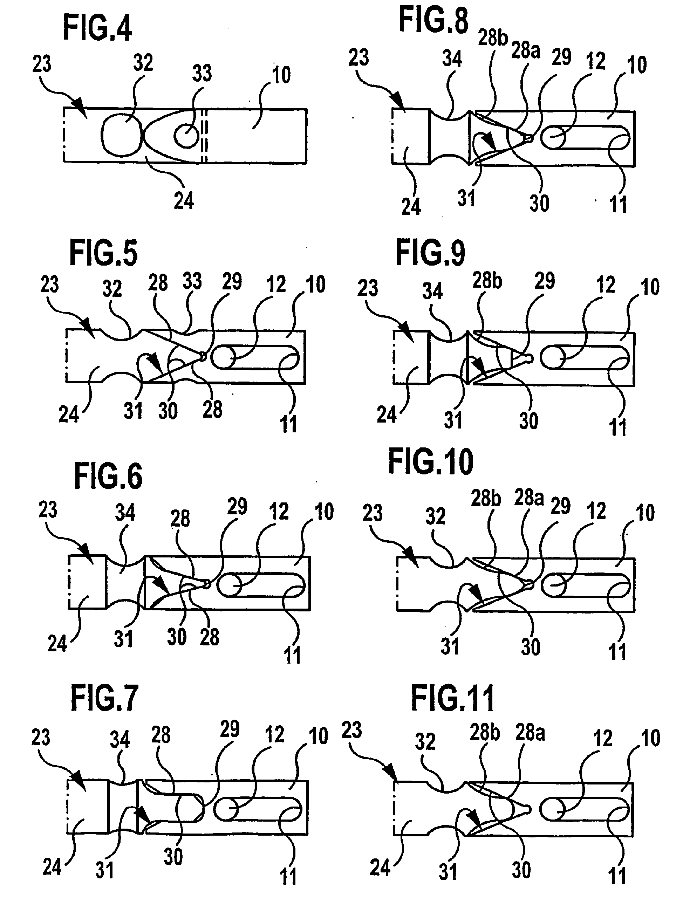

In diesem Anlagebereich sind auch abweichende geometrische Ausgestaltungen möglich, in den Figuren 6 bis 11 sind einige mögliche Abwandlungen beispielhaft dargestellt.

Bei dem Ausführungsbeispiel der Figur 6 sind die einander diametral gegenüberliegenden geradlinigen Nuten 32 ersetzt durch eine umlaufende Ringnut 34, außerdem ist der Einführtrichter 31 des Mitnehmers 10 an seiner Einführseite aufgeweitet, weist also einen größeren Keilwinkel auf. Dementsprechend sind auch die Anlageflächen 28 der Spitze 29 in diesem Bereich aufgeweitet, im Ausführungsbeispiel der Figur 6 mit bogenförmiger Kontur, so daß keine vollflächige Anlage in diesem Bereich mehr vorhanden ist. Die Drehmitnahme erfolgt bei diesem Ausführungsbeispiel nur im drehachsnahen Bereich von Spitze 29 und Mitnehmer 10.

Im Ausführungsbeispiel der Figur 7 ist eine ähnliche Ausgestaltung gewählt mit dem Unterschied, daß die Anlageflächen 28 und die komplementären Anlageflächen 30 nicht keil- oder V-förmig angeordnet sind, sondern parallel zueinander im Abstand verlaufen, und zwar parallel zur Drehachse des Werkzeuges 23.

In den Fällen der Figuren 1 bis 7 sind die Anlageflächen 28 und 30 so ausgebildet, daß sie im gekoppelten Zustand flächig aneinander anliegen und somit eine großflächige Drehmomentübertragung gewährleisten.

Bei den Ausführungsbeispielen der Figuren 8 bis 11 hingegen ergibt sich aufgrund der Form der Anlageflächen eine linienförmige Anlage zwischen den Anlageflächen.

Während bei diesen Ausgestaltungen die Anlageflächen 30 des Mitnehmers in gleicher Weise als ebene, keil- oder V-förmig verlaufende Flächen ausgebildet sind, weisen die Anlageflächen 28 des Schaftes 24 bei diesen Ausführungsbeispielen gebogene Konturen auf, und zwar gebogen in Längsrichtung der V-förmigen Schenkel, quer dazu sind die Anlageflächen 28 eben ausgebildet.

Beim Ausführungsbeispiel der Figur 8 weist die Anlagefläche 28 einen drehachsnahen konvexen Bereich 28a und einen drehachsfernen konkaven Bereich 28b auf, eine Anlage ergibt sich nur im konvexen Bereich 28a, und diese Anlage ist linienförmig.

Beim Ausführungsbeispiel der Figur 9 ist die Anlagefläche 28 über den größten Bereich konkav ausgebildet, nur der spitzennahe Endbereich ist abgeschrägt oder konvex ausgebildet, so daß sich an einem sehr kleinen Randbereich eine Anlage an der Anlagefläche 30 ergibt.

Ähnliche Konturen wie in Figur 8 sind bei den Ausführungsbeispielen der Figuren 10 und 11 gewählt, dabei weisen diese Ausführungsbeispiele keine Ringnut 34 auf, sondern einander gegenüberliegende Nuten 32.

## Patentansprüche

1. Kupplung für ein chirurgisches Drehantriebs-Handstück (1) mit einem in dem Handstück (1) gelagerten und von diesem drehend angetriebenen Werkzeug (23) mit einer hülsenförmigen drehangetriebenen Aufnahme (7) im Handstück (1), in die ein Schaft (24) des Werkzeuges (23) unter Ausbildung einer formschlüssigen Drehmitnahme einschiebbar ist, und mit mindestens einem radial in den Innenraum der Aufnahme (7) einschiebbaren Verriegelungskörper (15), der in einer eingeschobenen Verriegelungsstellung in einen Rücksprung des Schaftes (24) eingreift und diesen **dadurch** gegen axiale Verschiebung sichert, während er in einer radial ausgeschobenen Stellung aus dem Rücksprung austritt und damit eine axiale Verschiebung des Schaftes (24) in der Aufnahme (7) ermöglicht, **dadurch gekennzeichnet, daß** zur Ausbildung einer formschlüssigen Drehmitnahme in der hülsenförmigen Aufnahme (7) ein mit der Aufnahme (7) rotierender, gegenüber dieser axial verschieblicher Mitnehmer (10) angeordnet ist mit mindestens einer Anlagefläche (30) an seiner dem Werkzeug (23) zugewandten Seite, daß der Mitnehmer (10) unter der Wirkung einer Feder (13) in eine ausgeschobene Stellung verschiebbar ist, in der der oder die Verriegelungskörper (15) an ihm anliegen und **dadurch** in ihrer Freigabestellung gehalten werden, daß der Mitnehmer (10) mittels des an ihm anliegenden Schaftes (24) des Werkzeuges (23) gegen die Wirkung der Feder (13) in eine eingeschobene Stellung verschiebbar ist, in der der oder die Verriegelungskörper (15) in die Verriegelungsstellung eintreten und den Schaft (24) des Werkzeuges (23) gegen eine axiale Verschiebung sichern können, und daß der Schaft (24) des Werkzeuges (23) in dessen eingeschobener, durch den oder die Verriegelungskörper (15) gegen eine axiale Verschiebung gesicherten Stellung den Mitnehmer (10) gegen die Wirkung der Feder (13) derart in die Aufnahme (7) einschiebt, daß dessen Anlageflächen (30) an Anlageflächen (28) des Werkzeuges (23) unter Ausbildung einer Drehmitnahme anliegen.

2. Kupplung nach Anspruch 1, **dadurch gekennzeichnet, daß** die Anlageflächen (30; 28) des Mitnehmers (10) bzw. des Werkzeuges (23) flächig aneinander anliegen.

3. Kupplung nach Anspruch 1, **dadurch gekennzeichnet, daß** die Anlageflächen (30; 28) des Mitnehmers (10) bzw. des Werkzeuges (23) linienförmig aneinander anliegen.

4. Kupplung nach einem der voranstehenden Ansprüche, **dadurch gekennzeichnet, daß** die Anlageflächen (30) an einem Teil (10; 23) einen Einführtrichter (31) ausbilden und am anderen Teil (23; 10) eine Einführspitze (29).

5. Kupplung nach einem der voranstehenden Ansprüche, **dadurch gekennzeichnet, daß** der Mitnehmer (10) und das Werkzeug (23) im Bereich ihrer Anlageflächen (30 bzw. 28) spiegelsymmetrisch zu einer Spiegelebene sind, die durch die Drehachse der Aufnahme (7) und durch einen Durchmesser des eingesetzten Werkzeuges (23) aufgespannt wird.

6. Kupplung nach einem der voranstehenden Ansprüche, **dadurch gekennzeichnet, daß** der Mitnehmer (10) und das Werkzeug (23) jeweils zwei Anlageflächen (30 bzw. 28) aufweisen.

7. Kupplung nach Anspruch 6, **dadurch gekennzeichnet, daß** die Normalenvektoren der beiden Anlageflächen (28 bzw. 30) des Werkzeuges (23) und/oder des Mitnehmers (10) parallel zueinander verlaufen.

8. Kupplung nach einem der Ansprüche 6 oder 7, **dadurch gekennzeichnet, daß** die beiden Anlageflächen (30 bzw. 28) des Mitnehmers (10) und/oder des Werkzeuges (23) V-förmig angeordnet sind.

9. Kupplung nach einem der voranstehenden Ansprüche, **dadurch gekennzeichnet, daß** die Anlageflächen (30 bzw. 28) des Mitnehmers (10) und/oder des Werkzeuges (23) eben sind.

10. Kupplung nach einem der Ansprüche 1 bis 8, **dadurch gekennzeichnet, daß** die Anlageflächen (28) des Werkzeuges (23) oder des Mitnehmers (10) eine gebogene Kontur (28a, 28b) aufweisen.

11. Kupplung nach Anspruch 10, **dadurch gekennzeichnet, daß** die Kontur im drehachsnahen Bereich konvex ausgebildet und im drehachsfernen Bereich demgegenüber zurückgesetzt ist.

12. Kupplung nach Anspruch 11, **dadurch gekennzeichnet, daß** die Kontur im drehachsfernen Bereich konkav ausgebildet ist.

13. Kupplung nach einem der Ansprüche 4 bis 12, **dadurch gekennzeichnet, daß** der Öffnungswinkel des Einführtrichters (31) im drehachsfernen Bereich größer ist als im drehachsnahen Bereich.

14. Kupplung nach einem der Ansprüche 1 bis 7, **dadurch gekennzeichnet, daß** die Anlageflächen (28, 30) parallel zur Drehachse der Aufnahme (7) angeordnet sind.

15. Kupplung nach einem der Ansprüche 4 bis 14, **dadurch gekennzeichnet, daß** der Mitnehmer (10) an seinem dem Werkzeug (23) zugewandten, als Einführtrichter (31) ausgebildeten Ende keilförmig ist.

16. Kupplung nach Anspruch 15, **dadurch gekennzeichnet, daß** der Keilwinkel (β) des Mitnehmers (10) zwischen 60 und 90° liegt.

17. Kupplung nach einem der voranstehenden Ansprüche, **dadurch gekennzeichnet, daß** der den Verriegelungskörper (15) aufnehmende Rücksprung eine Umfangsnut (34) ist.

18. Kupplung nach einem der Ansprüche 1 bis 16, **dadurch gekennzeichnet, daß** der den Verriegelungskörper (15) aufnehmende Rücksprung eine quer zur Drehachse verlaufende, geradlinige Nut (32) an der Außenseite des Werkzeuges (23) ist.

19. Kupplung nach einem der Ansprüche 1 bis 16, **dadurch gekennzeichnet, daß** der Rücksprung eine randseitig geschlossene Vertiefung an der Außenseite des Werkzeuges (23) ist.

20. Kupplung nach Anspruch 19, **dadurch gekennzeichnet, daß** die Vertiefung die Form einer Kugelkalotte hat.

21. Kupplung nach einem der voranstehenden Ansprüche, **dadurch gekennzeichnet, daß** der Verriegelungskörper (15) in der eingeschobenen Verriegelungsstellung an der Kante des Rücksprunges (32, 34) punkt- oder linienförmig anliegt.

22. Kupplung nach Anspruch 21, **dadurch gekennzeichnet, daß** der Verriegelungskörper (15) eine Kugel ist und der Rücksprung (32, 34) eine bogenförmige Kontur aufweist, deren Radius geringfügig kleiner ist als der Radius des Verriegelungskörpers (15).

23. Kupplung nach einem der voranstehenden Ansprüche, **dadurch gekennzeichnet, daß** der Mitnehmer (10) in der Aufnahme (7) außenseitig einen Rücksprung (33) aufweist, in den der Verriegelungskörper (15) eintaucht, wenn sich der Mitnehmer (10) bei entspannter Feder (13) in seiner ausgeschobenen Stellung befindet.

24. Kupplung nach einem der voranstehenden Ansprüche, **dadurch gekennzeichnet, daß** die Ausschubbewegung des Mitnehmers (10) durch einen Anschlag (11, 12) der Aufnahme (7) begrenzt wird.

25. Kupplung nach einem der voranstehenden Ansprüche, **dadurch gekennzeichnet, daß** der Mitnehmer (10) einen zu seiner Verschieberichtung parallelen Schlitz (11) aufweist, durch den ein an der Aufnahme (7) festgelegter Mitnahmestift (12) hindurchragt.

26. Kupplung nach einem der voranstehenden Ansprüche, **dadurch gekennzeichnet, daß** der Verriegelungskörper (15) federnd in die Verriegelungsstellung verschoben wird.

27. Kupplung nach einem der voranstehenden Ansprüche, **dadurch gekennzeichnet, daß** mehrere Verriegelungskörper (15) vorgesehen sind.

28. Kupplung nach einem der voranstehenden Ansprüche, **dadurch gekennzeichnet, daß** der oder die Verriegelungskörper (15) Kugeln sind.

29. Kupplung nach einem der voranstehenden Ansprüche, **dadurch gekennzeichnet, daß** der Verriegelungskörper (15) in einer radialen Öffnung (14) der Aufnahme (7) verschieblich geführt ist.

30. Kupplung nach einem der voranstehenden Ansprüche, **dadurch gekennzeichnet, daß** die Aufnahme (7) zur Verschiebung des Verriegelungskörpers (15) von einer Verschiebehülse (16) mit einer Aufgleitfläche (17) für den Verriegelungskörper (15) umgeben und in Richtung auf ein radiales Einschieben des Verriegelungskörpers (15) federbelastet ist.

31. Kupplung nach Anspruch 30, **dadurch gekennzeichnet, daß** die Aufgleitfläche (17) mindestens in einem Teilabschnitt (17a) relativ zur Verschieberichtung der Verschiebehülse (16) geneigt ist.

32. Kupplung nach einem der voranstehenden Ansprüche, **dadurch gekennzeichnet, daß** der Schaft (24) Anlageflächen (28) aufweist zur drehfesten Anlage an Anlageflächen (30) des Mitnehmers (10), die in eine Einführspitze (29) einmünden, und daß sich an die Anlageflächen (28) auf deren der Einführspitze (29) abgewandten Seite mindestens ein Rücksprung (34; 32) für einen Verriegelungskörper (15) anschließt.

33. Kupplung nach Anspruch 32, **dadurch gekennzeichnet, daß** das Werkzeug (23) im Bereich seiner Anlageflächen (28) spiegelsymmetrisch zu einer Spiegelebene ist, die durch die Drehachse des Werkzeuges (23) und durch einen Durchmesser des eingesetzten Werkzeuges (23) aufgespannt wird.

34. Kupplung nach einem der Ansprüche 32 oder 33, **dadurch gekennzeichnet, daß** das Werkzeug (23) zwei Anlageflächen (28) aufweist.

35. Kupplung nach einem der Ansprüche 32 bis 34, **dadurch gekennzeichnet, daß** die Normalenvektoren der beiden Anlageflächen (28) des Werkzeuges (23) parallel zueinander verlaufen.

36. Kupplung nach Anspruch 34 oder 35, **dadurch gekennzeichnet, daß** die beiden Anlageflächen (28) des Werkzeuges (23) V-förmig angeordnet sind.

37. Kupplung nach einem der Ansprüche 32 bis 36 **dadurch gekennzeichnet, daß** die Anlageflächen (28) eben sind.

38. Kupplung nach einem der Ansprüche 32 bis 36, **dadurch gekennzeichnet, daß** die Anlageflächen (28) eine gebogene Kontur (28a, 28b) aufweisen.

39. Kupplung nach Anspruch 38, **dadurch gekennzeichnet, daß** die Kontur im drehachsnahen Bereich konvex ausgebildet und im drehachsfernen Bereich demgegenüber zurückgesetzt ist.

40. Kupplung nach Anspruch 38, **dadurch gekennzeichnet, daß** die Kontur im drehachsfernen Bereich konkav ausgebildet ist.

41. Kupplung nach einem der Ansprüche 33 bis 35, **dadurch gekennzeichnet, daß** die Anlageflächen (28) parallel zur Drehachse des Werkzeuges (23) angeordnet sind.

42. Kupplung nach einem der Ansprüche 32 bis 41, **dadurch gekennzeichnet, daß** der den Verriegelungskörper (15) aufnehmende Rücksprung eine Umfangsnut (34) ist.

43. Kupplung nach einem der Ansprüche 32 bis 41, **dadurch gekennzeichnet, daß** der den Verriegelungskörper (15) aufnehmende Rücksprung eine quer zur Drehachse verlaufende, geradlinige Nut (32) an der Außenseite des Werkzeuges (23) ist.

44. Kupplung nach einem der Ansprüche 32 bis 41, **dadurch gekennzeichnet, daß** der Rücksprung eine randseitig geschlossene Vertiefung an der Außenseite des Werkzeuges (23) ist.

45. Kupplung nach Anspruch 44, **dadurch gekennzeichnet, daß** die Vertiefung die Form einer Kugelkalotte hat.

## Claims

1. A coupling for a surgical rotary drive hand piece (1) including a tool (23) which is mounted in the hand piece (1) and driven thereby in rotary manner and incorporating a sleeve-shaped rotary driven seating (7) in the hand piece (1) into which a shank (24) of the tool (23) is adapted to be inserted so as to form an interlocking rotary drive means, and including at least one locking body (15) which is adapted to be inserted radially into the interior of the seating (7) and which, in an inserted locking position engages in a recess in the shank (24) and thereby secures it from axial displacement, whereas, in a radially withdrawn position, it leaves the recess and thus enables an axial displacement of the shank (24) in the seating (7), **characterised in that** a driver (10) having at least one contact surface (30) on the side thereof facing the tool (23) and which rotates with the sleeve (7) and is axially displaceable with respect thereto is arranged in the sleeve-shaped seating (7) for the purposes of forming an interlocking rotary drive means, **in that** the driver (10) is displaceable by the effect of a spring (13) into a pushed-out position in which the locking body or bodies (15) rest thereon and are thus held in their release position, **in that** the driver (10) is displaceable by means of the shank (24) of the tool (23) resting thereon against the effect of the spring (13) into a pushed-in position in which the locking body or bodies (15) enter the locking position and can secure the shank (24) of the tool (23) from axial displacement, and **in that** the shank (24) of the tool (23) in the pushed-in position thereof secured from axial displacement by the locking body or bodies (15) pushes the driver (10) against the effect of the spring (13) into the seating (7) in such a manner that its contact surfaces (30) rest against the contact surfaces (28) of the tool (23) so as to form a rotary drive means.

2. A coupling in accordance with Claim 1, **characterised in that** the respective contact surfaces (30; 28) of the driver (10) and the tool (23) engage each other with an areal contact.

3. A coupling in accordance with Claim 1, **characterised in that** the respective contact surfaces (30; 28) of the driver (10) and the tool (23) engage each other with a line contact.

4. A coupling in accordance with any of the preceding Claims, **characterised in that** the contact surfaces (30) on one part (10; 23) are in the form of a lead-in funnel (31) and on the other part (23; 10) are in the form of a lead-in tip (29).

5. A coupling in accordance with any of the preceding Claims, **characterised in that**, in the vicinity of their respective contact surfaces (30 and 28), the driver (10) and the tool (23) are mirror symmetrical with respect to a mirror plane which extends through the axis of rotation of the seating (7) and through a diameter of the inserted tool (23).

6. A coupling in accordance with any of the preceding Claims, **characterised in that** the driver (10) and the tool (23) each comprise two contact surfaces (30 and 28).

7. A coupling in accordance with Claim 6, **characterised in that** the respective normal vectors of the two contact surfaces (28 and 30) of the tool (23) and/or the driver (10) run in parallel with each other.

8. A coupling in accordance with either of Claims 6 or 7, **characterised in that** the respective two contact surfaces (30 and 28) of the driver (10) and/or the tool (23) are arranged to be V-shaped.

9. A coupling in accordance with any of the preceding Claims, **characterised in that** the respective contact surfaces (30 and 28) of the driver (10) and/or the tool (23) are flat.

10. A coupling in accordance with any of the Claims 1 to 8, **characterised in that** the contact surfaces (28) of the tool (23) or the driver (10) have a curved contour (28a, 28b).

11. A coupling in accordance with Claim 10, **characterised in that** the contour is convex in the region close to the rotational axis and is set back with respect thereto in the region remote from the rotational axis.

12. A coupling in accordance with Claim 11, **characterised in that** the contour is concave in the region remote from the rotational axis.

13. A coupling in accordance with any of the Claims 4 to 12, **characterised in that** the opening angle of the lead-in funnel (31) is larger in the region remote from the rotational axis than in the region close to the rotational axis.

14. A coupling in accordance with any of the Claims 1 to 7, **characterised in that** the contact surfaces (28, 30) are arranged in parallel with the axis of rotation of the seating (7).

15. A coupling in accordance with any of the Claims 4 to 14, **characterised in that** the driver (10) is wedge-shaped (31) at the lead-in funnel shaped end thereof facing the tool (23).

16. A coupling in accordance with Claim 15, **characterised in that** the wedge angle (β) of the driver (10) lies between 60 and 90°.

17. A coupling in accordance with any of the preceding Claims, **characterised in that** the recess accommodating the locking body (15) is a peripheral groove (34).

18. A coupling in accordance with any of the Claims 1 to 16, **characterised in that** the recess accommodating the locking body (15) is a straight-line groove (32) in the outer surface of the tool (23) which extends transversely with respect to the axis of rotation.

19. A coupling in accordance with any of the Claims 1 to 16, **characterised in that** the recess is a depression in the outer surface of the tool (23) which is closed at the edges thereof.

20. A coupling in accordance with Claim 19, **characterised in that** the recess is in the form of a ball joint.

21. A coupling in accordance with any of the preceding Claims, **characterised in that**, in the inserted locking position thereof, the locking body (15) engages the edge of the recess (32, 34) with a point or line contact.

22. A coupling in accordance with Claim 21, **characterized in that** the locking body (15) is a ball and the recess (32, 34) has an arc-shaped contour whose radius is slightly smaller than the radius of the locking body (15).

23. A coupling in accordance with any of the preceding Claims, **characterized in that** the driver (10) in the seating (7) comprises a recess (33) in its outer surface into which the locking body (15) extends when the driver (10) is in its pushed-out position with the spring (13) in its relaxed state.

24. A coupling in accordance with any of the preceding Claims, **characterised in that** the outward displacement of the driver (10) is limited by a stop means (11, 12) on the seating (7).

25. A coupling in accordance with any of the preceding Claims, **characterized in that** the driver (10) comprises a slot (11) which extends in parallel with its direction of displacement and through which there projects a driver pin (12) that is fixed to the seating (7).

26. A coupling in accordance with any of the preceding Claims, **characterised in that** the locking body (15) is shifted into the locking position in resilient manner.

27. A coupling in accordance with any of the preceding Claims, **characterised in that** a plurality of locking bodies (15) are provided.

28. A coupling in accordance with any of the preceding Claims, **characterised in that** the locking body or bodies (15) are balls.

29. A coupling in accordance with any of the preceding Claims, **characterised in that** the locking body (15) is guided in displaceable manner in a radial opening (14) of the seating (7).

30. A coupling in accordance with any of the preceding Claims, **characterised in that**, for the purposes of the displacement of the locking body (15), the seating (7) is surrounded by a displacement sleeve (16) incorporating a slide surface (17) for the locking body (15) and is spring loaded in the direction of radial insertion of the locking body (15).

31. A coupling in accordance with Claim 30, **characterised in that** the slide surface (17) is inclined over at least a partial section (17a) thereof relative to the direction of displacement of the displacement sleeve (16).

32. A coupling in accordance with any of the preceding Claims, **characterised in that** the shank (24) comprises contact surfaces (28) for making contact with contact surfaces (30) of the driver (10) in mutually non-rotatable manner and which merge into a lead-in tip (29), and **in that** at least one recess (34; 32) for a locking body (15) adjoins the contact surfaces (28) on the sides thereof remote from the lead-in tip (29).

33. A coupling in accordance with Claim 32, **characterised in that**, in the vicinity of its contact surfaces (28), the tool (23) is mirror symmetrical with respect to a mirror plane which extends through the axis of rotation of the tool (23) and through a diameter of the inserted tool (23).

34. A coupling in accordance with either of the Claims 32 or 33, **characterised in that** the tool (23) comprises two contact surfaces (28).

35. A coupling in accordance with any of the Claims 32 to 34, **characterised in that** the normal vectors of the two contact surfaces (28) of the tool (23) run in parallel with one another.

36. A coupling in accordance with Claim 34 or 35, **characterised in that** the two contact surfaces (28) of the tool (23) are arranged in the form of a V-shape.

37. A coupling in accordance with any of the Claims 32 to 36, **characterized in that** the contact surfaces (28) are flat.

38. A coupling in accordance with any of the Claims 32 to 36, **characterised in that** the contact surfaces (28) have a curved contour (28a, 28b).

39. A coupling in accordance with Claim 38, **characterised in that** the contour is convex in the region close to the rotational axis and is set back with respect thereto in the region remote from the rotational axis.

40. A coupling in accordance with Claim 38, **characterised in that** the contour is concave in the region remote from the rotational axis.

41. A coupling in accordance with any of the Claims 33 to 35, **characterised in that** the contact surfaces (28) are arranged in parallel with respect to the axis of rotation of the tool (23).

42. A coupling in accordance with any of the Claims 32 to 41, **characterised in that** the recess accommodating the locking body (15) is a peripheral groove (34).

43. A coupling in accordance with any of the Claims 32 to 41, **characterised in that** the recess accommodating the locking body (15) is a straight-line groove (32) in the outer surface of the tool (23) which extends transversely with respect to the axis of rotation.

44. A coupling in accordance with any of the Claims 32 to 41, **characterised in that** the recess is a depression in the outer surface of the tool (23) which is closed at the edges thereof.

45. A coupling in accordance with Claim 44, **characterised in that** the depression is in the form of a ball joint.

## Revendications

1. Accouplement pour poignée porte-outil chirurgicale (1) d'entraînement en rotation, comprenant un outil (23) monté dans la poignée porte-outil (1) et entraîné en rotation par celle-ci, comprenant également dans la poignée porte-outil (1), un logement de réception (7) en forme de fourreau, entraîné en rotation et dans lequel peut être emmanchée la queue d'outil (24) de l'outil (23) en réalisant une prise d'entraînement en rotation par complémentarité de formes, et comprenant en définitive au moins un corps de verrouillage (15) qui peut être engagé radialement dans l'espace intérieur du logement de réception (7) et qui, dans une position de verrouillage engagée vient en prise dans un décrochement en retrait de la queue d'outil (24) en bloquant ainsi celle-ci à l'encontre d'un coulissement axial, tandis que dans une position radialement extraite, il sort du décrochement en retrait en permettant ainsi un coulissement axial de la queue d'outil (24) dans le logement de réception (7), **caractérisé en ce que** pour réaliser une prise d'entraînement en rotation par complémentarité de formes, dans le logement de réception (7) en forme de fourreau est agencé un entraîneur (10) en rotation avec le logement de réception (7), pouvant coulisser axialement par rapport à celui-ci et comportant au moins une surface d'appui (30) sur son côté dirigé vers l'outil (23), **en ce que** l'entraîneur (10), sous l'action d'un ressort (13), peut coulisser dans une position sortie dans laquelle le ou les corps de verrouillage (15) s'appuient contre lui en les maintenant ainsi dans leur position de dégagement libre, **en ce que** l'entraîneur (10) peut, au moyen de la queue d'outil (24) de l'outil (23), qui s'y appuie, être déplacé par coulissement à l'encontre de l'action du ressort (13), dans une position rentrée dans laquelle le ou les corps de verrouillage (15) passent dans la position de verrouillage et peuvent bloquer la queue d'outil (24) de l'outil (23) à l'encontre d'un coulissement axial, et **en ce que** la queue d'outil (24) de l'outil (23) dans sa position rentrée et bloquée par les corps de verrouillage (15) à l'encontre d'un coulissement axial, fait coulisser l'entraîneur (10) à l'intérieur du logement de réception (7), à l'encontre de l'action du ressort (13), de façon telle que ses surfaces d'appui (30) s'appliquent contre les surfaces d'appui (28) de l'outil (23) en réalisant une prise d'entraînement en rotation.

2. Accouplement selon la revendication 1, **caractérisé en ce que** les surfaces d'appui (30 ; 28) de l'entraîneur (10) respectivement de l'outil (23) s'appliquent mutuellement les unes contre les autres selon un appui de surface.

3. Accouplement selon la revendication 1, **caractérisé en ce que** les surfaces d'appui (30 ; 28) de l'entraîneur (10) respectivement de l'outil (23) s'appliquent mutuellement les unes contre les autres selon un appui en forme de ligne.

4. Accouplement selon l'une des revendications précédentes, **caractérisé en ce que** les surfaces d'appui (30) forment sur une pièce (10 ; 23) un entonnoir d'introduction (31) et sur l'autre pièce (23 ; 10) une pointe d'introduction (29).

5. Accouplement selon l'une des revendications précédentes, **caractérisé en ce que** l'entraîneur (10) et l'outil (23) sont, dans une zone de leurs surfaces d'appui (30 respectivement 28), symétriques par rapport à un plan de symétrie, qui est défini par l'axe de: rotation du logement de réception (7) et par un diamètre de l'outil (23) mis en oeuvre.

6. Accouplement selon l'une des revendications précédentes, **caractérisé en ce que** l'entraîneur (10) et l'outil (23) présentent chacun deux surfaces d'appui (30 respectivement 28).

7. Accouplement selon la revendication 6, **caractérisé en ce que** les vecteurs normaux des deux surfaces d'appui (28 respectivement 30) de l'outil (23) et/ou de l'entraîneur (10) s'étendent parallèlement les uns aux autres.

8. Accouplement selon l'une des revendications 6 ou 7, **caractérisé en ce que** les deux surfaces d'appui (30 respectivement 28) de l'entraîneur (10) et/ou de l'outil (23) sont agencées en forme de V.

9. Accouplement selon l'une des revendications précédentes, **caractérisé en ce que** les surfaces d'appui (30 respectivement 28) de l'entraîneur (10) et/ou de l'outil (23) sont planes.

10. Accouplement selon l'une des revendications 1 à 8, **caractérisé en ce que** les surfaces d'appui (28) de l'outil (23) ou de l'entraîneur (10) présentent un contour (28a, 28b) courbe.

11. Accouplement selon la revendication 10, **caractérisé en ce que** le contour est de configuration convexe dans la zone proche de l'axe de rotation et est au contraire en retrait par rapport à cela dans la zone éloignée de l'axe de rotation.

12. Accouplement selon la revendication 11, **caractérisé en ce que** le contour est de configuration concave dans la zone éloignée de l'axe de rotation.

13. Accouplement selon l'une des revendications 4 à 12, **caractérisé en ce que** l'angle d'ouverture de l'entonnoir d'introduction (31) est plus grand dans la zone éloignée de l'axe de rotation que dans la zone proche de l'axe de rotation.

14. Accouplement selon l'une des revendications 1 à 7, **caractérisé en ce que** les surfaces d'appui (28, 30) sont agencées parallèlement à l'axe de rotation du logement de réception (7).

15. Accouplement selon l'une des revendications 4 à 14, **caractérisé en ce que** l'entraîneur (10) est en forme de coin à son extrémité qui est dirigée vers l'outil (23) et est réalisée en forme d'entonnoir d'introduction (31).

16. Accouplement selon la revendication 15, **caractérisé en ce que** l'angle de coin (β) de l'entraîneur (10) se situe entre 60 et 90°.

17. Accouplement selon l'une des revendications précédentes, **caractérisé en ce que** le décrochement en retrait recevant le corps de verrouillage (15), est une rainure périphérique (34).

18. Accouplement selon l'une des revendications 1 à 16, **caractérisé en ce que** le décrochement en retrait recevant le corps de verrouillage (15), est une rainure rectiligne (32), qui s'étend transversalement à l'axe de rotation, sur le côté extérieur de l'outil (23).

19. Accouplement selon l'une des revendications 1 à 16, **caractérisé en ce que** le décrochement en retrait est un creux à bord fermé, sur le côté extérieur de l'outil (23).

20. Accouplement selon la revendication 19, **caractérisé en ce que** le creux à une forme de calotte sphérique.

21. Accouplement selon l'une des revendications précédentes, **caractérisé en ce que** le corps de verrouillage (15), dans la position rentrée de verrouillage, s'appuie en un point ou selon une ligne sur le bord du décrochement en retrait (32, 34).

22. Accouplement selon la revendication 21, **caractérisé en ce que** le corps de verrouillage (15) est une bille, et le décrochement en retrait (32, 34) présente un contour courbe dont le rayon est très légèrement inférieur au rayon du corps de verrouillage (15).

23. Accouplement selon l'une des revendications précédentes, **caractérisé en ce que** l'entraîneur (10) dans le logement de réception (7) présente du côté extérieur, un décrochement en retrait (33) dans lequel s'engage le corps de verrouillage (15), lorsque l'entraîneur (10) se trouve dans sa position extraite, pour un ressort (13) détendu.

24. Accouplement selon l'une des revendications précédentes, **caractérisé en ce que** le coulissement d'extraction de l'entraîneur (10) est limité par une butée (11, 12) du logement de réception (7).

25. Accouplement selon l'une des revendications précédentes, **caractérisé en ce que** l'entraîneur (10) présente une fente (11) qui est parallèle à sa direction de coulissement et est traversée par une goupille d'entraînement (12) fixée au logement de réception (7).

26. Accouplement selon l'une des revendications précédentes, **caractérisé en ce que** le corps de verrouillage (15) est déplacé de manière élastique dans la position de verrouillage.

27. Accouplement selon l'une des revendications précédentes, **caractérisé en ce que** sont prévus plusieurs corps de verrouillage (15).

28. Accouplement selon l'une des revendications précédentes, **caractérisé en ce que** le ou les corps de verrouillage (15) sont des billes.

29. Accouplement selon l'une des revendications précédentes, **caractérisé en ce que** le corps de verrouillage (15) est guidé en coulissement dans une ouverture radiale (14) du logement de réception (7).

30. Accouplement selon l'une des revendications précédentes, **caractérisé en ce que** pour faire coulisser le corps de verrouillage (15), le logement de réception (7) est entouré par une douille de coulissement (16) qui comprend une surface de rampe (17) pour le corps de verrouillage (15), et est chargée par ressort en direction d'une rentrée radiale du corps de verrouillage (15).

31. Accouplement selon la revendication 30, **caractérisé en ce que** la surface de rampe (17) est, au moins sur un tronçon partiel (17a), incliné par rapport à la direction de coulissement de la douille de coulissement (16).

32. Accouplement selon l'une des revendications précédentes, **caractérisé en ce que** la queue d'outil (24) comporte des surfaces d'appui (28) pour l'appui fixe en rotation contre des surfaces d'appui (30) de l'entraîneur (10), qui débouchent dans une pointe d'introduction (29), et **en ce qu'**aux surfaces d'appui (28), sur leur côté éloigné de la pointe d'introduction (29), se raccorde au moins un décrochement en retrait (34 ; 32) pour un corps de verrouillage (15).

33. Accouplement selon la revendication 32, **caractérisé en ce que** l'outil (23), dans la zone de ses surfaces d'appui (28), est symétrique par rapport à un plan de symétrie, qui est défini par l'axe de rotation de l'outil (23) et par un diamètre de l'outil (23) mis en oeuvre.

34. Accouplement selon l'une des revendications 32 ou 33, **caractérisé en ce que** l'outil (23) présente deux surfaces d'appui (28).

35. Accouplement selon l'une des revendications 32 à 34, **caractérisé en ce que** les vecteurs normaux des deux surfaces d'appui (28) de l'outil (23) s'étendent parallèlement les uns aux autres.

36. Accouplement selon la revendication 34 ou la revendication 35, **caractérisé en ce que** les deux surfaces d'appui (28) de l'outil (23) sont agencées en forme de V.

37. Accouplement selon l'une des revendications 32 à 36, **caractérisé en ce que** les surfaces d'appui (28) sont planes.

38. Accouplement selon l'une des revendications 32 à 36, **caractérisé en ce que** les surfaces d'appui (28) présentent un contour (28a, 28b) courbe.

39. Accouplement selon la revendication 38, **caractérisé en ce que** le contour est de configuration convexe dans la zone proche de l'axe de rotation et est en retrait par rapport à cela dans la zone éloignée de l'axe de rotation.

40. Accouplement selon la revendication 38, **caractérisé en ce que** le contour est de configuration concave dans la zone éloignée de l'axe de rotation.

41. Accouplement selon l'une des revendications 33 à 35, **caractérisé en ce que** les surfaces d'appui (28) sont agencées parallèlement à l'axe de rotation de l'outil (23).

42. Accouplement selon l'une des revendications 32 à 41, **caractérisé en ce que** le décrochement en retrait recevant le corps de verrouillage (15), est une rainure périphérique (34).

43. Accouplement selon l'une des revendications 32 à 41, **caractérisé en ce que** le décrochement en retrait recevant le corps de verrouillage (15), est une rainure rectiligne (32), qui s'étend transversalement à l'axe de rotation, sur le côté extérieur de l'outil (23).

44. Accouplement selon l'une des revendications 32 à 41, **caractérisé en ce que** le décrochement en retrait est un creux à bord fermé, sur le côté extérieur de l'outil (23).

45. Accouplement selon la revendication 44, **caractérisé en ce que** le creux à une forme de calotte sphérique.
